# EUROPEAN PATENT APPLICATION

(11) **EP 4 083 194 A1**
(43) Date of publication of application: **02.11.2022**
(21) Application number: 20894351.4
(22) Date of filing: 18.11.2020
(51) Int. Cl.: C12N 5/077

(54) **COMPOSITION FOR MEDIUM**

(30) Priority: 25.11.2019 JP 2019211991
(71) Applicant: Kataoka Corporation, Kyoto-shi, Kyoto 601-8203 (JP); Kyoto Prefectural Public University Corporation, Kyoto-shi, Kyoto 602-8566 (JP)
(72) Inventor: DAI Ping, Kyoto-shi Kyoto 602-8566 (JP); TAKEDA Yukimasa, Kyoto-shi Kyoto 602-8566 (JP); HARADA Yoshinori, Kyoto-shi Kyoto 602-8566 (JP); MATSUMOTO Junichi, Kyoto-shi Kyoto 601-8203 (JP)
(74) Representative: Vossius & Partner Patentanwälte Rechtsanwälte mbB
(86) International application number: PCT/JP2020/042899
(87) International publication number: WO 2021/106698

(57) **Abstract**

The present invention is primarily directed to provide a new composition for a medium which can be used for differentiation induction from somatic cells to brown adipocytes.

The present invention can include, for example, a composition for a medium used in differentiation induction from somatic cells to brown adipocytes, wherein the composition comprises the following seven components: a thyroid hormone receptor agonist, a glucocorticoid receptor agonist, a phosphodiesterase inhibitor, insulin, an ascorbic acid derivative, albumin, and an antibiotic.

According to the present invention, direct differentiation induction from somatic cells to brown adipocytes can be effectively performed. In addition, according to the present invention, it is possible to effectively maintain brown adipocytes.

## Description

### TECHNICAL FIELD

### (Cross-Reference to Related Applications)

This application claims the benefit of Japanese Patent Application No. 2019-211991, filed November 25, 2019 with the Japanese Patent Office. The Japanese application is hereby incorporated by reference for all purposes as if the entirety of its application documents (specification, claims, drawings, and abstract) were expressly set forth herein.

The present invention belongs to the technical field of regenerative medicine. The present invention relates in its technical field to a composition for a medium which can be used in differentiation induction from somatic cells to brown adipocytes, and a composition for a medium for maintaining brown adipocytes induced to differentiate.

### BACKGROUND OF THE INVENTION

In recent years, methods (direct reprogramming) for converting somatic cells, such as fibroblasts, directly into other cell types by low molecular weight compounds without gene transfer into somatic cells have been actively studied. For example, in Patent Document 1, several low molecular weight compounds such as an ALK5 inhibitor and an ALK6 inhibitor are combined, and in the presence thereof, somatic cells, particularly human fibroblasts, are cultured, and are directly converted into brown adipocytes and the like. As in the invention of Patent Document 1, it is very beneficial if conventional fibroblasts can be directly converted into other cell types such as brown adipocytes with a low molecular weight compound which is easy to obtain. For example, autologous fibroblasts can be conveniently used to produce other autologous cell types, thereby increasing their applications in regenerative medicine. In addition, cells for the development of new pharmaceuticals can be easily produced as experimental materials.

In Non-Patent Document 1, as in Patent Document 1, human skin fibroblasts are cultured in the presence of a combination of several low molecular weight compounds and brown adipocytes are directly induced from such fibroblasts. Specifically, in the invention of Non-Patent Document 1, human skin fibroblasts are cultured in the presence of low molecular weight compounds such as SB431542 (ALK5 inhibitor), LDN193189 (ALK2/3 inhibitor), dorsomorphin (ALK2/3/6 inhibitor), forskolin (cAMP inducer), and rosiglitazone (PPARγ agonist), and brown adipocytes are directly induced from such fibroblasts. The combination of said low molecular weight compounds is also disclosed in Patent Document 1.

The differentiation induction from somatic cells to brown adipocytes and the like as described above is always performed by culturing under a medium containing various other compounds, nutrients and the like. The medium can include, for example, conventional mediums such as MEMs (minimal essential medium), DMEM (Dulbecco's Modified Eagle's Medium), DMEM/F12, or a modified medium thereof, and these are commercially available. Furthermore, in general, appropriate components (serum, proteins, amino acids, saccharides, vitamins, fatty acids, antibiotics, and the like) are added to these medium in consideration of cells to be induced, and the cell differentiation is carried out. Since serum such as fetal bovine serum (FBS) contains components necessary for cell growth such as hormones, growth factors, and adhesion factors, it is often carried out to induce differentiation by adding serum. In the inventions of Patent Document 1 and Non-Patent Document 1, differentiation induction is carried out under a medium to which FBS is added.

### PRIOR ART DOCUMENTS

### PATENT DOCUMENT

Patent Document 1: WO 2018/062269

### NON-PATENT DOCUMENT

Non-Patent Document 1: Takeda Y., Harada Y., Yoshikawa T., and Dai P., Sci. Rep., 7,4304 (2017)

### SUMMARY OF THE INVENTION

### PROBLEM TO BE SOLVED BY THE INVENTION

The present invention is primarily directed to provide a new composition for a medium which can be used in differentiation induction from somatic cells to brown adipocytes. It is also an object of the present invention to provide a new composition for a medium for maintaining brown adipocytes induced to differentiate.

### MEANS FOR SOLVING THE PROBLEMS

As a result of intensive studies, the present inventors have found that the above problem can be solved by a composition for a medium containing certain low molecular weight compounds, and have completed the present invention.

The present invention can include, for example, the following embodiments.
[1] A composition for a differentiation induction medium used in differentiation induction from somatic cells to brown adipocytes, comprising the following seven components:
   - a thyroid hormone receptor agonist
   - a glucocorticoid receptor agonist
   - a phosphodiesterase inhibitor
   - insulin
   - an ascorbic acid derivative
   - albumin, and
   - an antibiotic.
[2] The composition for a differentiation induction medium according to the [1] mentioned above, wherein the differentiation induction is performed directly without introducing a gene into a somatic cell.
[3] The composition for a differentiation induction medium according to the [1] or [2] mentioned above, wherein the thyroid hormone receptor agonist is triiodothyronine, the glucocorticoid receptor agonist is dexamethasone, the phosphodiesterase inhibitor is isobutylmethylxanthine, the ascorbic acid derivative is L-ascorbic acid 2-phosphate, the albumin is an albumin to which linoleic acid and oleic acid are bound or a fatty acid-unbound albumin, or the antibiotic is penicillin and/or streptomycin.
[4] The composition for a differentiation induction medium according to any one of the [1] to [3] mentioned above, further comprising a selective PPAKγ agonist, or a selective PPAKγ agonist and a cAMP inducer and/or a BMP7.
[5] The composition for a differentiation induction medium according to the [4] mentioned above, wherein the selective PPAKγ agonist is rosiglitazone or the cAMP inducer is forskolin.
[6] A differentiation induction medium, comprising the composition for a differentiation induction medium according to any one of the [1] to [5] mentioned above, and being serum-free.
[7] The differentiation induction medium according to the [6] mentioned above, prepared by adding the composition for a differentiation induction medium according to any one of the [1] to [5] mentioned above to a DMEM medium.
[8] The differentiation induction medium according to the [7] mentioned above, wherein the DMEM medium is a high glucose DMEM medium.
[9] The differentiation induction medium according to the [7] or [8] mentioned above, wherein the DMEM medium contains L-glutamine.
[10] The composition for a differentiation induction medium or the differentiation induction medium according to any one of the [1] to [9] mentioned above, wherein the somatic cell is a fibroblast.
[11] A brown adipocyte produced by using the composition for a differentiation induction medium or the differentiation induction medium according to any one of the [1] to [10] mentioned above.
[12] A composition for a maintenance medium for maintaining brown adipocytes produced by differentiation induction from somatic cells, comprising the following five components:
   - a selective PPAKγ agonist
   - a glucocorticoid receptor agonist
   - insulin
   - albumin, and
   - an antibiotic.
[13] The composition for a maintenance medium according to the [12] mentioned above, wherein the selective PPAKγ agonist is rosiglitazone, the glucocorticoid receptor agonist is dexamethasone, the albumin is an albumin to which linoleic acid and oleic acid are bound or a fatty acid-unbound albumin, or the antibiotic is penicillin and/or streptomycin.
[14] A maintenance medium, comprising the composition for a maintenance medium according to the [12] or [13] mentioned above, and being serum-free.
[15] The maintenance medium according to the [14] mentioned above, prepared by adding the composition for a maintenance medium according to the [12] or [13] mentioned to a DMEM medium.
[16] The maintenance medium according to the [15] mentioned above, wherein the DMEM medium is a high-glucose DMEM medium.
[17] The maintenance medium according to the [15] or [16] mentioned above, wherein the DMEM medium contains L-glutamine and pyruvate.
[18] The composition for a maintenance medium or the maintenance medium according to any one of the [12] to [17] mentioned above, wherein the somatic cell is a fibroblast.

### EFFECT OF THE INVENTION

According to the present invention, differentiation induction from somatic cells to brown adipocytes can be effectively performed. Moreover, according to the present invention, it is possible to effectively maintain brown adipocytes induced to differentiate from somatic cells.

### BRIEF DESCRIPTION OF THE DRAWINGS

[Figure 1] Expression levels of Ucp1 genes are represented. Symbol 1 denotes Comparative Example 6 (control), Symbol 2 denotes Example 1, Symbol 3 denotes Comparative Example 1, Symbol 4 denotes Comparative Example 2, Symbol 5 denotes Comparative Example 3, Symbol 6 denotes Comparative Example 4, and Symbol 7 denotes Comparative Example 5. The vertical axis represents the relative value of mRNA amount relative to the expression amount of the internal standard gene (Tbp).
[Figure 2] Expression levels of Fabp4 genes are represented. Each symbol and the vertical axis are the same as in Figure 1.
[Figure 3] Expression levels of Ucp1 genes are represented. Symbol 1 denotes Example 2, Symbol 2 denotes Example 3, Symbol 3 denotes Example 4, Symbol 4 denotes Comparative Example 7, Symbol 5 denotes Example 5, Symbol 6 denotes Comparative Example 8, and Symbol 7 denotes Example 6. The vertical axis represents the relative value of mRNA amount relative to the expression amount of the internal standard gene (Tbp).
[Figure 4] Expression levels of Fabp4 genes are represented. Each symbol and the vertical axis are the same as in Figure 3.
[Figure 5] Photographs of cultured cells directly induced from fibroblasts with combinations of compounds (Example 7) and said cells which were incubated with maintenance medium (Example 8), and photographs of said cells in which mitochondria (red), UCP1 protein (green) and cell nucleus (blue) were immuno-stained. Since Figure 5 is monochrome, the colors red, green, and blue are not displayed, but the colors are displayed in the original photograph of Figure 5.
[Figure 6] Expression levels of the genes are represented. In each of the figures, the vertical axis represents the mRNA amount of the gene relative to that of the internally standard gene (Tbp). Numerals on the horizontal axis indicate time (week).
[Figure 7] Expression levels of the genes are represented. In each of the figures, the vertical axis represents the mRNA amount of the gene relative to the that of the internally standard gene (Tbp). Numerals on the horizontal axis indicate time (week).
[Figure 8] Expression levels ofUcp1 genes are represented. Symbol 1 denotes a control, Symbol 2 denotes Example 11, Symbol 3 denotes Example 12, Symbol 4 denotes Comparative Example 13, and Symbol 5 denotes Example 14. The vertical axis represents the relative value of mRNA amount relative to the expression level of the internal standard gene (Tbp).
[Figure 9] Expression levels of Ckmt 1 genes are represented. Each symbol and the vertical axis are the same as in Figure 8.

### EMBODIMENT FOR CARRYING OUT THE PRESENT INVENTION

Hereinafter, the present invention will be described in detail.

### 1 Composition for Differentiation Induction Medium

A composition for a differentiation induction medium according to the present invention (hereinafter, referred to as "the present invention induction composition") is a composition for a medium used in differentiation induction from somatic cells to brown adipocytes, comprising the following seven components:
- a thyroid hormone receptor agonist
- a glucocorticoid receptor agonist
- a phosphodiesterase inhibitor
- insulin
- an ascorbic acid derivative
- albumin, and
- an antibiotic.

Each of the above-mentioned components may be used in one kind or combination of two or more kinds thereof in each case.

Differentiation induction from somatic cells to brown adipocytes may be performed either by what is called direct programming, in which the somatic cells are not subjected to an artificial gene transfer, but are directly converted by small molecular compounds, or by a method in which the somatic cells are subjected to an artificial gene transfer. Among these, it is preferable that the present invention induction composition is used for differentiation induction which is directly performed without introducing genes into somatic cells.

Moreover, the present invention can include a differentiation induction medium containing the present invention induction composition, and being serum-free (hereinafter, referred to as "the present invention induction medium").

Here, the term "serum-free" refers to the state of being substantially free of unadjusted or unpurified serum, and in the present invention, even if a differentiation induction medium or a maintenance medium is contaminated with a purified component derived from blood or animal tissue, the medium is referred to as a serum-free differentiation induction medium or maintenance medium, so long as it does not substantially contain an unadjusted or unpurified serum.

The term "brown adipocyte" in the present invention refers to those which are artificially induced to differentiate from somatic cells, have many mitochondria and many fat droplets, and express UCP 1 (uncoupling protein-lgenes). The brown adipocyte can also include what is called human beige cells and bright cells, induced from white adipocytes or their progenitor cells. What is called chemical compound-induced brown adipocytes, ciBAs, are also included, which are produced by a method in which direct conversion is performed by low molecular weight compounds without transfer of artificial genes into somatic cells. In addition to terminally differentiated brown adipocyte, the brown adipocyte can also include progenitor cells destined to differentiate into brown adipocytes.

The present invention induction composition or the present invention induction medium may further contain a selective PPAKγ agonist, or a selective PPAKγ agonist and a cAMP inducer and/or a BMP7, as an active component for differentiation induction into brown adipocytes. Among them, it is preferable to further contain a selective PPAKγ agonist, and it is more preferable to further contain two components of a selective PPAKγ agonist and a cAMP inducer, or three components of a selective PPAKγ agonist, a cAMP inducer, and a BMP7.

### 1.1 Somatic cell

Cells of an organism can be classified into somatic and germ cells. Any somatic cell can be used as a starting material in the present invention process. The somatic cell is not particularly limited, and may be either a primary cell taken from a living body or a cell from an established cell line. Somatic cells at various stages of differentiation, e.g., terminally differentiated somatic cells (e.g., fibroblasts, umbilical vein endothelial cells (HUVEC), hepatic cells (Hepatocytes), bile duct cells (Biliary cells), pancreatic alpha cells (Pancreatic α cells), pancreatic acinar cells (Acinar cells), pancreatic ductal glandular cells (Ductal cells), small intestinal crypt cells (Intestinal crypt cells), etc.), somatic cells on the way to terminal differentiation (e.g., mesenchymal stem cells, neural stem cells, endodermal progenitor cells, etc.), or such somatic cells like iPS cells that have been initialized to acquire pluripotency and somatic cells that have differentiated or are in the process of differentiation from iPS cells, can be used in the present invention process. The somatic cells that can be used in the present invention process include any somatic cells, for example, cells of the hematopoietic system (various lymphocytes, macrophages, dendritic cells, bone marrow cells, etc.), cells derived from organs (hepatocytes, splenocytes, pancreatic cells, kidney cells, lung cells, etc.), cells of the muscle tissue system (skeletal muscle cells, smooth muscle cells, myoblasts, cardiomyocytes, etc.), fibroblasts, neurons, osteoblasts, chondrocytes, endothelial cells, interstitial cells, adipocytes (white adipocytes, etc.), embryonic stem cells (ES cells), etc. The present invention process can also be applied to the precursor cells of these cells and cancer cells. Preferably, fibroblasts can be used.

Fibroblasts that can be used in the present invention are not particularly limited, and examples of the fibroblasts can include skin fibroblasts (Dermal Fibroblasts), aortic outer membrane fibroblasts (Adventitial Fibroblasts), cardiac fibroblasts (Cardiac Fibroblasts), pulmonary fibroblasts (Pulmonary Fibroblasts), uterine fibroblasts (Uterine Fibroblasts), chorionic mesenchymal fibroblasts (Villous Mesenchymal Fibroblasts), all of which are primary cell components constituting connective tissues in various tissues and organs, and are cells producing collagen fibers.

Examples of the source of the above-mentioned somatic cells include, but are not limited to, humans, mammals other than humans, and animals other than mammals (birds, reptiles, amphibians, fishes, and the like). As a source of somatic cells, humans and mammals other than humans are preferred, and humans are particularly preferred. When brown adipocytes are induced to differentiate for the purpose of administration to humans, preferably, somatic cells harvested from a donor having a matched or similar type of histocompatibility antigen to the recipient can be used. Somatic cells harvested from the recipient itself may be used.

### 1.2 Each component

### 1.2.1 Thyroid hormone receptor agonist

The thyroid hormone receptor agonist is not particularly limited, as long as it binds to thyroid hormone receptors and has a hormone-like effect; and can include, for example, the following compounds, and preferably include triiodothyronine and thyroxine.

Triiodothyronine (CAS No.: 6893-02-3)

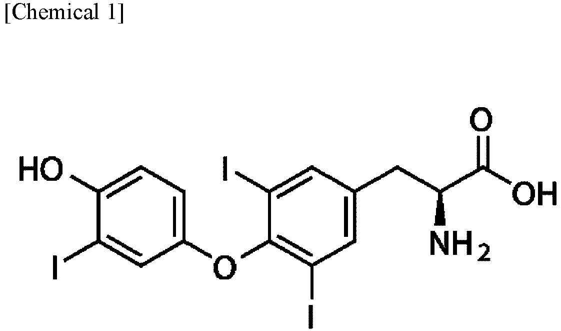

3,5-diiodothyronine dihydrate (CAS No.: 18835-59-1)
Tiratricol (CAS No.: 51-24-1)
Liothyronine Sodium (CAS No.: 55-06-1)
L-thyroxine (CAS No.: 51-48-9)

The concentration of the thyroid hormone receptor agonist varies depending on the kind of the agonist, the somatic cell used, and the like, but is not particularly limited and may be appropriately determined, and may be used in a range of, for example, 1 nmol/L to 10 µmol/L, preferably 0.01 µmol/L to 1 µmol/L.

### 1.2.2 Glucocorticoid receptor agonist

The glucocorticoid receptor agonist is not particularly limited, as long as it binds to glucocorticoid receptors and has a glucocorticoid-like effect; and can include, for example, the following compounds, and preferably include, dexamethasone, hydrocortisone, corticosterone, and cortisone.

Dexamethasone (CAS No.: 50-02-2)

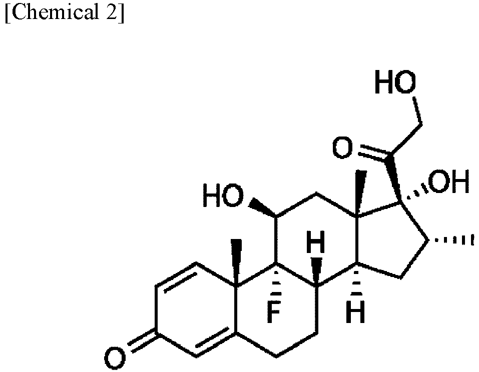

Dexamethasone Sodium Phosphate (CAS No.: 55203-24-2)
Dexamethasone acetate (CAS No.: 1177-87-3)
Meprednisone (CAS No.: 1247-42-3)
Fluocinolone acetonide (CAS No.: 67-73-2)
Budesonide (CAS No.: 51333-22-3)
Desoximetasone (CAS No.: 382-67-2)
Protopanaxatriol (CAS No.: 34080-08-5)
Triamcinolone (CAS No.: 124-94-7)
Beclomethasone dipropionate (CAS No.: 5534-09-8)
Prednisolone (CAS No.: 50-24-8)
Hydrocortisone (CAS No.: 50-23-7)
Deflazacort (CAS No.: 14484-47-0)
Triamcinolone acetonide (CAS No.: 76-25-5)
Fluocinonide (CAS No.: 356-12-7)
Methylprednisolone sodium succinate (CAS No.: 2921-57-5)
Ciclesonide (CAS No.: 126544-47-6)
Flumetasone (CAS No.: 2135-17-3)
Fluorometholone (CAS No.: 426-13-1)
Betamethasone dipropionate (CAS No.: 5593-20-4)
Betamethasone Valerate (CAS No.: 2152-44-5)
Mometasone Furoate (CAS No.: 83919-23-7)
Fluticasone propionate (CAS No.: 80474-14-2)
Cortisone acetate (CAS No.: 50-04-4)
Cortodoxone (CAS No.: 152-58-9)
Roteprednol (CAS No.: 82034-46-6)
Betamethasone (CAS No.: 378-44-9)
Methylprednisolone (CAS No.: 83-43-2)
Corticosterone (CAS No.: 50-22-6)
Cortisone (CAS No.: 53-06-5)

The concentration of the glucocorticoid receptor agonist varies depending on the kind of the agonist, the somatic cell used, and the like, but is not particularly limited and may be appropriately determined, and may be used in a range of, for example, 0.01 µmol/L to 30 µmol/L, preferably 0.1 (µmol/L to 10 (µmol/L.

### 1.2.3 Phosphodiesterase inhibitor

The phosphodiesterase inhibitor is not particularly limited, as long as it can increase cAMP concentration in cells by inhibiting phosphodiesterase or PDE, particularly PDE3, PDE4, PDE5 therein; and can include, for example, the following compounds, and preferably include isobutylmethylxanthine (IBMX) and rolipram.

Isobutylmethylxanthine (CAS No.: 28822-58-4)

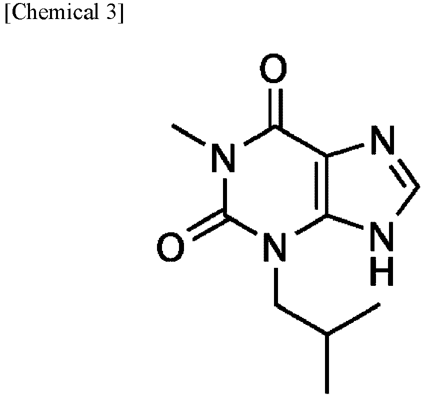

Roflumilast (CAS No.: 162401-32-3)
Sildenafil (CAS No.: 139755-83-2)
Tadalafil (CAS No.: 171596-29-5)
Vardenafil Hydrochloride Hydrate (CAS No.: 330808-88-3)
Pimobendan (CAS No.: 74150-27-9)
GSK256066 (CAS No. :801312-28-7)
Rolipram (CAS No.: 61413-54-5)
Apremilast (CAS No.: 608141-41-9)
Cilostazol (CAS No.: 73963-72-1)
Milrinone (CAS No.: 78415-72-2)
Abanafil (CAS No.: 330784-47-9)
Rolipram (CAS No.: 85416-73-5)
Fenspiride (CAS No.: 5053-08-7).
Udenafil (CAS No.: 268203-93-6)
Cilostamide (CAS No.: 68550-75-4)
Ibudilast (CAS No.: 50847-11-5)
Luteolin (CAS No.: 491-70-3)

The concentration of the phosphodiesterase inhibitor varies depending on the kind of the inhibitor, the somatic cell used, and the like, but is not particularly limited and may be appropriately determined, and may be used in a range of, for example, 1 µmol/L to 1 mmol/L, preferably 10 µmol/L to 0.5 mmol/L.

### 1.2.4 Ascorbic acid derivative

The ascorbic acid derivative is not particularly limited, as long as it is vitamin C, L-ascorbic acid, or derivatives thereof improved in instability of vitamin C. Specific examples thereof can include the following compounds such as vitamin C or L-ascorbic acid, and L-ascorbic acid 2-phosphate, 2PAA.

Sodium L-ascorbyl-2-phosphate (CAS No.: 66170-10-3)

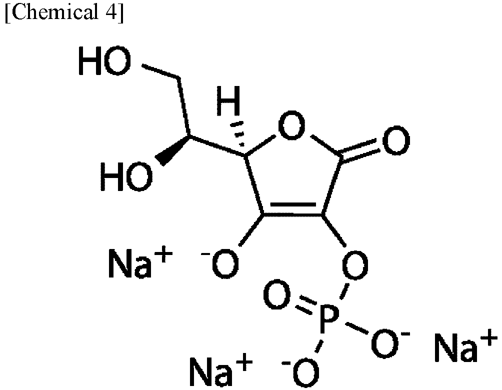

Ascorbyl palmitate (CAS No.: 137-66-6)
Sodium Ascorbate (CAS No.: 134-03-2)

The concentration of the ascorbic acid derivative varies depending on the kind of the derivative, the somatic cell used, and the like, but is not particularly limited and may be appropriately determined, and may be used in a range of, for example, 1 µg/mL to 1 mg/mL, preferably 10 µg/mL to 250 (µg/mL.

### 1.2.5 Insulin, fatty acid-bound albumin, and antibiotic

Insulin is a peptide hormone secreted from β-cells of the pancreas, and in the present invention, either insulin derived from animals or from humans may be used. The insulin may be chemically synthesized insulin or recombinant insulin produced by gene recombination technology.

In the present invention, human insulin is preferably used.

The concentration of insulin varies depending on the kind of the insulin, the somatic cell used, and the like, but is not particularly limited and may be appropriately determined, and may be used preferably in a range of 100 ng/mL to 20 (µg/mL.

The albumin may be a fatty acid-bound albumin to which a fatty acid is bound, or a fatty acid-unbound albumin, a fatty acid-free albumin, in which a fatty acid is removed from an albumin. Examples of the albumin can include an albumin purified from serum of an animal (e.g., bovine), the purified albumin thereof to which a known fatty acid is bound; and a recombinant albumin expressed by using animal cells or plant cells. In particular, a recombinant albumin which has been confirmed to be free of animal-derived components or a purified albumin to which a known fatty acid is bound is preferred. The albumin derived from humans is also preferred.

Examples of the fatty acid to be bound to albumin can include representative saturated and unsaturated fatty acids such as linoleic acid, oleic acid, linolenic acid, palmitic acid, arachidonic acid, eicosapentaenoic acid or EPA, docosahexaenoic acid or DHA, and the like. The mixed fatty acid of linoleic acid and oleic acid is preferred.

Specific examples of the fatty acid-bound albumin can include an albumin purified from serum of an animal, a linoleic acid and an oleic acid co-bound albumin. In the present invention, a commercially available fatty acid-bound bovine serum albumin (e.g., L9655: manufactured by Sigma-Aldrich Co., Ltd.) can be used.

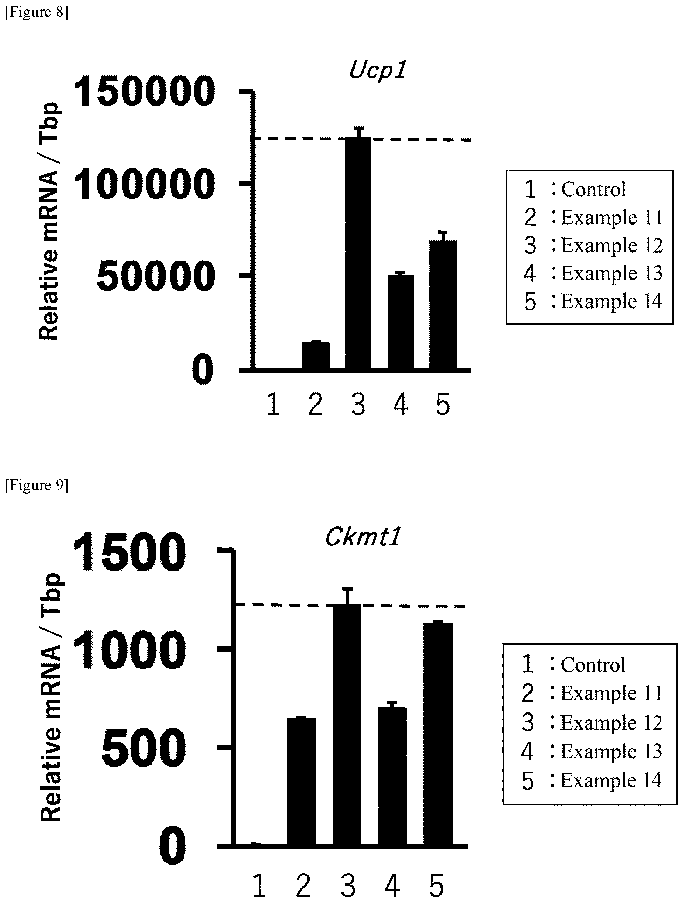

Note that in the experimental results mentioned later, brown adipocytes induced by using a fatty acid-unbound albumin had much higher expression levels of Ucp 1 genes than those induced by using a fatty acid-bound albumin (see Figures 8 and 9). In addition, in induction from fibroblasts to brown adipocytes, it was preferable not to add fatty acids. These are not observed in mesenchymal stem cell-derived adipocytes with high adipogenic activity.

The concentration of albumin varies depending on the kind of the albumin, the somatic cell used, and the like, but is not particularly limited and may be appropriately determined, and may be used in a range of, for example, 0.1 mg/mL to 10 mg/mL, preferably 0.5 mg/mL to 4 mg/mL.

The antibiotic is not particularly limited, as long as it can be used for cell culture of animals, and can include, for example, β-lactam antibiotics such as penicillins, cephems, and monobactams; aminoglycosides, macrolides, tetracyclines, peptides, nucleic acids, and polyenes. Specific examples can include actinomycin D, amphotericin B, ampicillin, antimycin A, bafilomycin A1, bleomycin, carbenicillin, chloramphenicol, concanamycin B, erythromycin, G418, gentamycin, hygromycin, kanamycin, mitomycin C, neomycin, oligomycin, penicillin, puromycin, rapamycin, streptomycin, tetracycline, tobramycin, and valinomycin. Among these, in the present invention, the combination of penicillin and streptomycin is suitable.

The content of the antibiotic varies depending on the kind of the antibiotic, the somatic cell used, and the like, but is not particularly limited and may be appropriately determined, and can be used, for example, in a range of 10 units/mL to 1000 units/mL, and preferably in a range of 50 units/mL to 200 units/mL. Depending on the type of the antibiotic, the antibiotic can be used in a range of 10 µg/mL to 1000 µg/mL, preferably in a range of 50 µg/mL to 200 (µg/mL.

### 1.2.6 Selective PPAKγ agonist

PPAKγ (Peroxisome proliferator-activated Receptor γ) is one of the proteins belonging to the nuclear receptor superfamily and is a transcription factor that controls gene expression by forming heterodimers with retinoid X receptors (RXRs) and binding to the promoter regions of target genes. There are three subtypes of PPARs: α, δ (β), and γ, and the present invention uses an agonist that selectively acts on γ .

Although not particularly limited in the present invention, examples of the selective PPAKγ agonist include the following compounds or salts thereof. Preference is given to selective PPAKγ agonists of the thiazolidine system, in particular rosiglitazone, pioglitazone, and troglitazone.

Rosiglitazone (CAS No.: 122320-73-4)

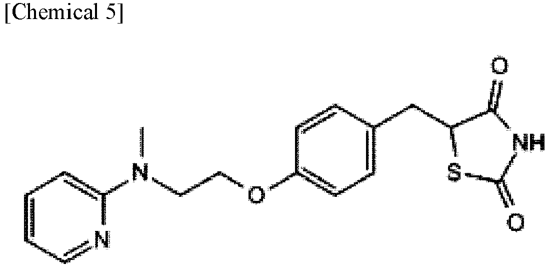

Rosiglitazone Maleate (CAS No.: 155141-29-0)
Ciglitazone (CAS No.: 74772-77-3)
Edaglitazone (CAS No.: 213411-83-7)
GW1929 Hydrochloride (CAS No.: 1217466-21-1)
nTZDpa (CAS No: 118414-59-8)
Pioglitazone Hydrochloride (CAS No.: 112529-15-4)
S26948 (CAS No: 353280-43-0)
Troglitazone (CAS No.: 97322-87-7)
Baraglitazone (CAS No.: 199113-98-9)
Rivoglitazone (CAS No.: 185428-18-6)

The concentration of the selective PPAKγ agonist varies depending on the kind of the agonist, the somatic cell used, and the like, but is not particularly limited and may be appropriately determined, and may be used in a range of, for example, 0.1 µmol/L to 20 µmol/L, preferably 0.5 µmol/L to 5 (µmol/L.

### 1.2.7 cAMP inducer

cAMP (cyclic adenosine monophosphate) is a second messenger that is involved in a variety of intracellular signaling events. The cAMP is produced intracellularly by cyclization of adenosine triphosphate (ATP) by adenylyl cyclase (adenylate cyclase).

Although not particularly limited in the present invention, examples of the cAMP inducer (adenylate cyclase activator) include forskolin (CAS No.: 66575-29-9), and forskolin derivatives (e.g., JP 2002-348243) and the following compounds. Preferably, forskolin can be used.

Forskolin (CAS No.: 66428-89-5)

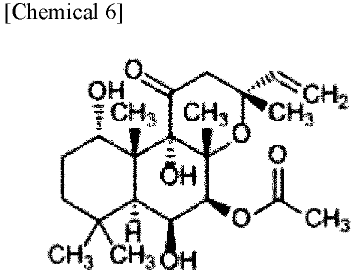

Isoproterenol (CAS No.: 7683-59-2)
NKH477 (CAS No: 138605-00-2)
PACAP1-27 (CAS No: 127317-03-7)
PACAP1-38 (CAS No: 137061-48-4)

The concentration of the cAMP inducer varies depending on the kind of the inducer, the somatic cell used, and the like, but is not particularly limited and may be appropriately determined, and may be used in a range of, for example, 0.1 µmol/Lto 100 µmol/L, preferably 0.5 µmol/L to 30 µmol/L.

### 1.2.8 BMP7

BMP (Bone Morphogenetic Protein: osteogenic proteins) 7 is one of the BMP families. Human BMP7 consists of 117 amino acid residues and activates the BMP signaling pathway by binding to cell surface receptors as a homodimer. Six types from BMP2 to BMP7 are classified as TGFb superfamilies, and are respectively encoded by distinct genes. In the present invention, either of a BMP7 derived from a human or a BMP7 derived from an animal may be used, and a recombinant thereof may be used. Among them, it is preferable to use a BMP7 derived from a human.

The concentration of the BMP7 varies depending on the kind of the BMP7, the somatic cell used, and the like, but is not particularly limited and may be appropriately determined, and may be used in a range of, for example, 0.1 ng/mL to 200 ng/mL, preferably 1 ng/mL to 50 ng/mL.

### 1.3 Somatic cell culture

The present invention induction composition can be used as a composition for differentiation induction from somatic cells to brown adipocytes. The present invention induction composition or the present invention induction medium can be also used as a medium for differentiation induction from somatic cells to brown adipocytes.

Culturing of somatic cells can be carried out in a conventional method, for example, by adding somatic cells to a medium in which the present invention induction composition containing components effective for differentiation induction into brown adipocytes is added to a basal medium prepared by mixing components necessary for cell culturing, or to the present invention induction medium, which is serum-free, containing components effective for differentiation induction into brown adipocytes, and appropriately selecting a temperature and other conditions according to the type of somatic cells used.

The component effective for differentiation induction into brown adipocytes is required to be contained in a concentration effective for producing brown adipocytes, and the concentration thereof may be appropriately determined by a person skilled in the art. The basal medium mentioned above may be selected from known or commercially available media. For example, general basal media such as MEM (Eagle's Minimum Essential Medium), GMEM (Glasgow's Minimum Essential Medium), DMEM (Dulbecco's Modified Eagle's Medium), Ham's F-12, DMEM/F12, RPMI1640, and IMDM (Iscov's Modified Dulbecco's Medium), or a medium modified therewith can be used as a basal medium.

One embodiment of the present invention induction medium may include a differentiation induction medium produced by adding the present invention induction composition to a DMEM medium. The "DMEM medium" is a basal medium well known to those skilled in the art which is widely used for culturing mammalian cells, and basically contains amino acids, mineral salts, D-glucose, and vitamins. In the present invention, it is preferable to use a high glucose DMEM medium or a DMEM medium containing L-glutamine and pyruvate.

In some cases, brown adipocytes can be produced by differentiation induction from somatic cells by one-step culturing using the present invention induction composition or the present invention induction medium. Further, selecting such somatic cells that are easy to culture as the somatic cells to be used makes it possible to increase the number of the somatic cells to reach an almost confluent state in advance of differentiation induction to produce brown adipocytes

As a culture condition, a general condition of cell culture can be selected. Conditions of 37°C and 5% CO₂, and the like are exemplified. It is preferred to change the medium at appropriate intervals during culture (preferably once every one to five days, and more preferably once every two to three days).

For culturing somatic cells, a cell culture container such as a plate, a dish, a cell culture flask, a cell culture bag, or the like can be used. Note that, as the cell culture bag, one having gas permeability is suitable. A large culture vessel may be used when large amounts of cells are required. The culture can be carried out in either an open system or a closed system, but when the purpose is administration or the like of the obtained brown adipocytes to a human, it is preferable to carry out the culture in a closed system.

### 1.4 Detection, confirmation and the like of brown adipocytes

The brown adipocytes produced by the present invention induction composition or the present invention induction medium can be detected, confirmed, and separated using, for example, morphological changes of cells, characteristic properties of brown adipocytes, and specific markers.

Adipocytes accumulate fat within cells. Thus, adipocytes can be detected by staining of intracellular fat using Oil Red O.

Specific markers for brown adipocytes can include, but are not limited to, UCP1, EVOL3 (Elongation of very long chain fatty acid protein 3), PGC1A (PPAR gamma coactivator 1-alpha), PRDM16 (PRD1-BF1-RIZ1 homologous domain containing 16), CITED 1 (CBP/p300 interacting transactivator with Glu/Asp rich carboxy-terminal domain 1), and the like. UCP1 is a kind of uncoupling proteins.

Quarantine methods (detection with antibodies) can be used to detect specific markers, but detection can be performed with quantitation of mRNA quantities of protein molecules. Antibodies that recognize specific markers of brown adipocytes are also useful in isolating and purifying brown adipocytes obtained by the present invention induction composition or the present invention induction medium.

The brown adipocytes produced by the present invention induction composition or the present invention induction medium can be used, for example, for tissue repair or the like after surgical treatment. Brown adipocytes produced by the present invention induction composition or the present invention induction medium can be used to produce pharmaceutical compositions for tissue repair and the like. In addition, transplantation and administration of brown adipocytes to the living body are expected to have effects on the improvement of the metabolism of the living body, the prevention of obesity, and the like.

When the brown adipocytes are used as pharmaceutical compositions, they may be formulated in a form suitable for administration to an individual by conventional methods, such as by mixing brown adipocytes with a pharmaceutically acceptable carrier. Examples of the carrier can include physiological saline and distilled water for injection which is made isotonic by the addition of glucose or other auxiliary agents (e.g., D-sorbitol, D-mannitol, sodium chloride, and the like). Further, buffering agents (e.g., phosphate buffer, sodium acetate buffer), painless agents (e.g., benzalkonium chloride, procaine hydrochloride, and the like), stabilizers (e.g., human serum albumin, polyethylene glycol, and the like), preservatives, antioxidants, and the like may be formulated.

The brown adipocytes produced by the present invention induction composition or the present invention induction medium may also be formulated in compositions in combination with other cells or components effective in enhancing the functional manifestation and the engraftment of brown adipocytes.

Further, the brown adipocytes produced by the present invention induction composition or the present invention induction medium can also be used for screening pharmaceutical candidate compounds which acts on brown adipocytes and for evaluating the safety of pharmaceutical candidate compounds. Thus, the brown adipocytes produced by the present invention induction composition or the present invention induction medium can be used to screen for agents that enhance the glucose-lipid metabolism or heat production function of brown adipocytes. The pharmaceutical candidate compounds for the screening include, for example, existing medicines such as so-called lifestyle-related disease therapeutic drugs, metabolic disease therapeutic drugs, and other library drugs. The food ingredients include, for example, nutrients of a wide variety of foods such as vitamins, saccharides, amino acids, minerals, and the like; ingredients of functional foods; and low molecular weight compounds, proteins, lipids, and the like in Chinese medicines, herbs, or other foods.

The above-described screening can be carried out, for example, by adding a test substance to the brown adipocytes produced by the present invention induction composition or the present invention induction medium, or the cells under production thereof, and evaluating the degree of increase in browning tendency in the cells by the test substance. In such evaluations, for example, the expression levels of Ucp1 gene and Fabp4 gene can be quantified by, for example, real-time PCR, and then the ratio thereof can be used as an indicator of the tendency of browning. Pharmaceutical candidate compounds and food ingredients that enhance the browning tendency analyzed by this method can be potential candidate compounds capable of increasing brown adipocytes (beige cells) in the human body.

### 2 Composition for maintenance medium

The composition for maintenance medium according to the present invention (hereinafter referred to as "the present invention maintenance composition ") is a composition for a medium for maintenance of brown adipocytes induced to differentiate from somatic cells, comprising the following five components:
- a selective PPAKγ agonist
- a glucocorticoid receptor agonist
- insulin
- albumin, and
- an antibiotic.

Each of the above-mentioned components may be used in one kind or two or more kinds in combination.

The present invention can include a maintenance medium containing the present invention maintenance composition and being serum-free (hereinafter referred to as "the present invention maintenance medium").

Here, the terms, "somatic cell", "differentiation induction", "serum-free", "brown adipocyte", "selective PPAKγ agonist", "glucocorticoid receptor agonist", "insulin", "albumin", "antibiotic", and other components are the same as mentioned above.

The present invention maintenance composition can be used as a composition for maintaining brown adipocytes produced by differentiation induction from somatic cells. The present invention maintenance composition or the present invention maintenance medium can be also used as a medium for maintaining brown adipocytes produced by differentiation induction from somatic cells.

Maintaining the brown adipocytes can be carried out in a conventional method, for example, by adding the brown adipocytes to a medium, in which the present invention maintenance composition is added to a basal medium prepared by mixing components necessary for culturing of cells, or the present invention maintenance medium, which is serum-free, and by culturing the brown adipocytes by appropriately selecting a temperature and other conditions according to the type of the brown adipocytes to be maintained.

The basal medium mentioned above may be selected from a known or commercially available medium. For example, general basal media such as MEM (Eagle's Minimum Essential Medium), GMEM (Glasgow's Minimum Essential Medium), DMEM (Dulbecco's Modified Eagle's Medium), Ham's F-12, DMEM/F12, RPMI1640, and IMDM (Iscov's Modified Dulbecco's Medium); or a medium modified therewith can be used as a basal medium.

The present invention maintenance medium can include, as an embodiment, a maintenance medium produced by adding the present invention maintenance composition to a DMEM medium. In the present invention, it is preferable to use a high glucose DMEM or a DMEM containing L-glutamine and pyruvate.

As a culture condition for the maintenance, a general condition of cell culture can be selected. Conditions of 37 °C and 5% CO₂, and the like are exemplified. It is preferred to change the medium at appropriate intervals during culture (preferably once every one to five days, and more preferably once every two to three days).

For culturing and maintaining the brown adipocytes, a cell culture container such as a plate, a dish, a cell culture flask, a cell culture bag, or the like can be used. Note that, as the cell culture bag, one having gas permeability is suitable. A large culture vessel may be used when large amounts of cells are required. The culture can be carried out in either an open system or a closed system, but when the purpose is administration or the like of the obtained brown adipocytes to a human, it is preferable to carry out the culture in a closed system.

### EXAMPLE

Hereinafter, the present invention will be specifically illustrated by way of Examples, Comparative Examples, and Test Examples, but the present invention is not limited to the scope of the Examples and the like.

### [Test Example A] Investigation of Composition for Differentiation Induction Medium or Differentiation Induction Medium

### (1) Cell culture

The human fibroblasts used as the material were purchased from DS Pharma Biomedical Co., Ltd. The fibroblasts are derived from 38-year-old human skin.

The above human fibroblasts were seeded in 35 mm dishes coated with gelatin (Cat#: 190-15805, manufactured by FUJIFILM Wako Pure Chemical Co.) at 5 × 10⁴, and cultured in DMEM medium (Cat#: 11965092; manufactured by Gibco) supplemented with 10% fetal bovine serum (FBS), 100 U/mL penicillin, 100 µg/mL streptomycin until 80% to 90% confluency at 37°C, 5% CO₂.

### (2) Differentiation induction from human fibroblasts to brown adipocytes

The medium used for direct conversion into brown adipocytes was the serum-free medium, and was prepared by adding each component of Table 1 to a DMEM medium (Cat#: 11965092; manufactured by Gibco), so as to have the said final concentration, in combinations of the compounds shown in Table 2 (Example 1 and Comparative Examples 1 to 6).

**[Table 1]**

| Components | Final conc. | |
|---|---|---|
| Triiodothyronine (T6397, by Merck) | 0.1 µmol/L | Thyroid hormone receptor agonist |
| Dexamethasone (041-18863, by FUJIFILM Wako Pure Chemical Co.) | 0.5 µmol/L | Glucocorticoid receptor agonist |
| IBMX (3-isobutyl-1-methylxanthine) (095-03413, by the same company above) | 0.03 µmol/L | Phosphodiesterase inhibitor |
| Insulin (093-06351, by the same company above) | 6.6 µg/mL | |
| L-ascorbic acid 2-phosphate (323-44822, by the same company above) | 100 µg/mL | Ascorbic acid derivative |
| Linoleic acid-Oleic acid-Albumin (L9655, by Merck) | 1 mg/mL | Fatty acid-bound albumin |
| Penicillin-Streptomycin (15070063, by Gibco) | 100 U/mL | Antibiotic |

**[Table 2]**

| Compounds | Example 1 | Comp. Ex. 1 | Comp. Ex. 2 | Comp. Ex. 3 | Comp. Ex. 4 | Comp. Ex. 5 | Comp. Ex. 6 |
|---|---|---|---|---|---|---|---|
| Triiodothyronine | + | - | + | + | + | + | - |
| Dexamethasone | + | + | - | + | + | + | - |
| IBMX | + | + | + | - | + | + | - |
| Insulin | + | + | + | + | - | + | - |
| 2PAA | + | + | + | + | + | - | - |
| Linoleic acid-Oleic acid - Albumin | + | + | + | + | + | + | - |
| Penicillin-Streptomycin | + | + | + | + | + | + | - |

In Table 2, the mark "+" indicates that the component was added to the medium, and the mark "-" indicates that the component was not added to the medium.

In addition, each of the compounds shown in Table 3 was added to the above medium so as to have the final concentration.

**[Table 3]**

| Compounds | Final conc. |
|---|---|
| Rosiglitazone (184-02651, by FUJIFILM Wako Pure Chemical Co.) | 1 µmol/L |
| Forskolin (063-02193, by the same company above) | 7.5 µmol/L |
| BMP7 (026-19171, by the same company above) | 20 ng/mL |

After the human fibroblasts reached 80% to 90% confluency, the medium in the dish was replaced with the serum-free medium mentioned above and the human fibroblasts were cultured in the medium for three weeks. The medium was changed every three days.

### (3) RNA isolation and QRT-PCR

To quantify gene expression, total RNAs were extracted from fibroblasts treated with the respective compounds using FastGene (registered trademark) RNA basic kit (manufactured by Nippon Genetics Co., Ltd.), followed by reverse-transcription by ReverTraAce (registered trademark) PCR RT Master Mix with gDNA Remover (manufactured by Toyobo Co., Ltd.), and then the real-time PCR analysis was performed by Power SYBR Green PCR Master Mix (Thermo Fisher Scientific). Three reactions were carried out per level under the following conditions: 40 cycles of reactions at 95°C for 10 minutes, at 95 °C for 15 seconds, and at 60°C for 60 seconds, in order. All results were normalized to the quantity of Tbp mRNA. Primers used in QRT-PCR are shown in Table 4 below.

**[Table 4]**

| Genes | Sense primer | Antisense primer |
|---|---|---|
| *Tbp* | ACTACGGGGTTATCACCTGTGAG (Sequence No.1) | GTGCAGGAGTAGGCCACATTAC (Sequence No.2) |
| *Ucp1* | TCTACGACACGGTCCAGGAG | GAATACTGCCACTCCTCCAGTC |
| | (Sequence No.3) | (Sequence No.4) |
| *Fabp4* | GCCAGGAATTTGACGAAGTCA (Sequence No.5) | CCCATTTCTGCACATGTACCAG (Sequence No.6) |

### (4) Results

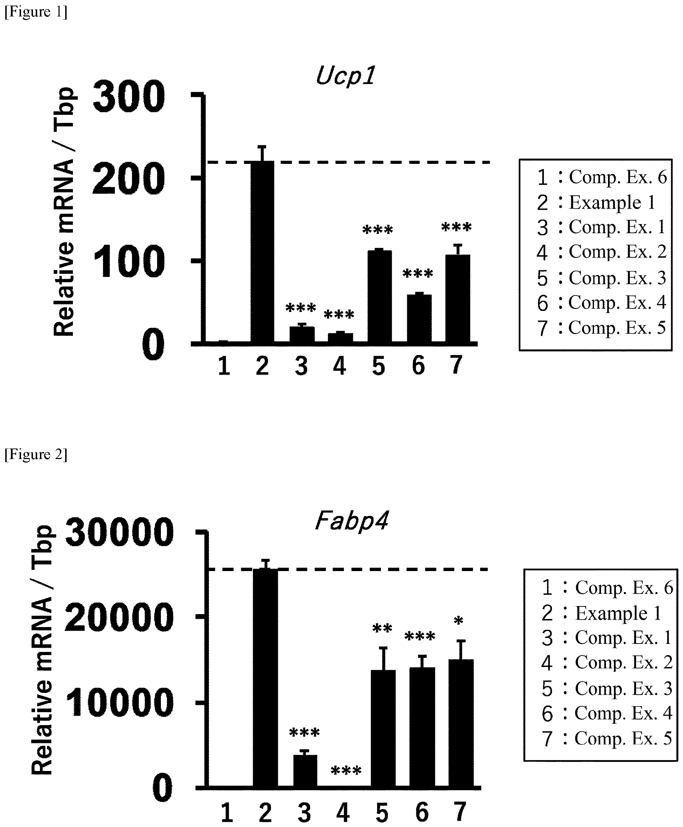

The experimental results are shown in Figures 1 and 2. Note that throughout the figures, the data indicate the mean ± SD (n=3), the marks "^{∗∗∗}", "^{∗∗}" and "^{∗}" indicate significant differences at p<0.001, p<0.01 and p<0.05, respectively, by Student t-test.

The composition for a medium added in Example 1 is the combination of triiodothyronine, dexamethasone, IBMX, insulin, 2PAA, linoleic acid-oleic acid-albumin, and penicillin/streptomycin. Then, in Comparative Examples 1 to 5, any one of the components of triiodothyronine, dexamethasone, IBMX, insulin, and 2PAA was excluded from this combination. In addition, in Comparative Example 6, none of the components shown in Table 1 was added as a medium component.

As shown in Figures 1 and 2, whereas both Ucp1 and Fabp4 are remarkably expressed in Example 1, their expression is poor in Comparative Examples. In particular, in the combination of the components of Comparative Examples 1 and 2, little Ucp1 is expressed.

Thus, it has been shown that the combination of Example 1 is preferred as the component of the medium for differentiation induction from human fibroblasts to brown adipocytes.

### [Test Example B] Investigation of Composition for Maintenance Medium, or Maintenance Medium

### (1) Cell culture

The same human fibroblasts as those in Test Example A mentioned above were cultured under the same conditions until 80% to 90% confluency.

### (2) Differentiation induction from human fibroblasts to brown adipocytes

The serum-free medium (serum-free brown adipocyte-inducing medium) used for differentiation induction into brown adipocytes was prepared by adding the same components as those in Example 1 to a DMEM medium (Cat#: 11965092; manufactured by Gibco). Hereinafter, the prepared medium is referred to as "SFBAM". Each of the components shown in Table 3 was further added to SFBAM.

After the human fibroblasts reached 80% to 90% confluency, the medium in the dish was replaced with SFBAM, a serum-free induction medium, containing all the compounds shown in Table 3 and the human fibroblasts were cultured in the medium for three weeks. The medium was changed every three days. Each of the induced cell was cultured in a serum-free maintenance medium containing all the components shown in Table 5 (Example 2) or in a serum-free maintenance medium containing at least rosiglitazone (Examples 3 to 6, Comparative Examples 7 and 8) for another one week to make chemically induced brown adipocyte-like cells (ciBAs) mature. The maintenance medium was prepared by adding each of the components shown in Table 5 below to a DMEM medium (Cat#: 11965092; manufactured by Gibco Co., Ltd.) in the combinations shown in Table 6 so as to have the respective final concentration.

**[Table 5]**

| Components | Final conc. |
|---|---|
| Rosiglitazone (184-02651, by FUJIFILM Wako Pure Chemical Co.) | 1 µmol/L |
| Forskolin (063-02193, by the same company above) | 7.5 µmol/L |
| BMP7 (026-19171, by the same company above) | 20 ng/mL |
| Triiodothyronine (T6397, by Merck) | 0.1 µmol/L |
| Dexamethasone (041-18863, by FUJIFILM Wako Pure Chemical Co.) | 0.5 µmol/L |
| 3-isobutyl-1-methylxanthine (IBMX) (095-03413, by the same company above) | 0.03 mmol/L |
| Insulin (093-06351, by the same company above) | 6.6 (µg/mL |
| L-ascorbic acid 2-phosphate (323-44822, by the same company above) | 100 µg/mL |
| Linoleic acid-Oleic acid-Albumin (L9655, by Merck) | 1 mg/mL |
| Penicillin-Streptomycin (15070063, by Gibco) | 100 Unit/mL |

**[Table 6]**

| Components | Example 2 | Example 3 | Example 4 | Example 5 | Example 6 | Comp. Ex. 7 | Comp. Ex. 8 |
|---|---|---|---|---|---|---|---|
| Rosiglitazone | + | + | + | + | + | + | + |
| Forskolin | + | - | - | - | - | - | - |
| BMP7 | + | - | - | - | - | - | - |
| Triiodothyronine | + | + | - | + | + | + | + |
| Dexamethasone | + | + | + | + | + | - | + |
| IBMX | + | + | + | - | + | + | + |
| Insulin | + | + | + | + | + | + | - |
| 2PAA | + | + | + | + | - | + | + |
| Linoleic acid-Oleic acid - Albumin | + | + | + | + | + | + | + |
| Penicillin-Streptomycin | + | + | + | + | + | + | + |

In Table 6, the mark "+" indicates that the component was added to the medium, and the mark "-" indicates that the component was not added to the medium.

### (3) RNA isolation and QRT-PCR

RNA isolation and PCR analysis were performed in the same manner in Test Example A mentioned above.

### (4) Results

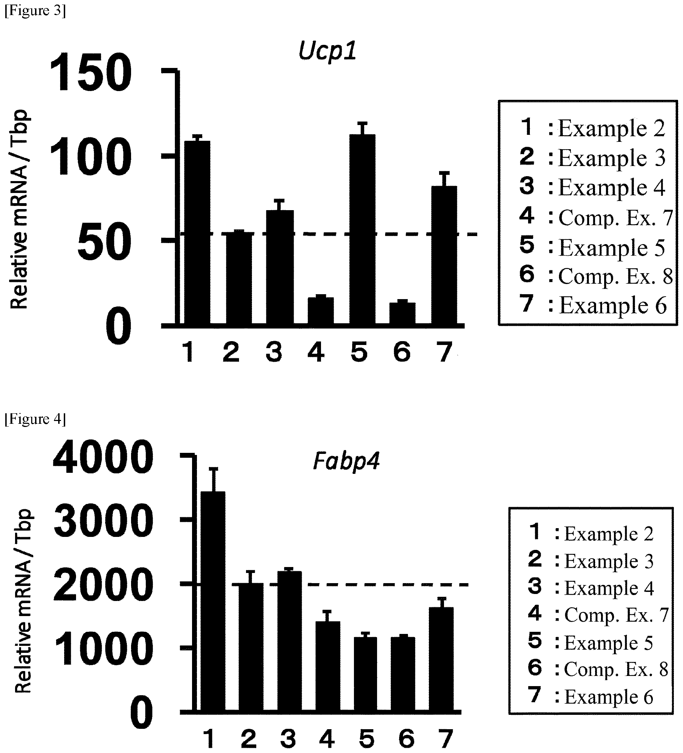

The experimental results are shown in Figures 3 and 4.

The composition for a medium added in Examples 2 and 3 was the combination of triiodothyronine, dexamethasone, IBMX, insulin, 2PAA, linoleic acid-oleic acid-albumin, and penicillin/streptomycin. Then, in Examples 4 to 6 and Comparative Examples 7 and 8, any one of the components of triiodothyronine, dexamethasone, IBMX, insulin, and 2PAA was excluded from this combination.

As shown in Figures 3 and 4, Ucp1 specific for brown adipocytes was markedly expressed in the case of serum-free maintenance medium of Examples 2 to 6. In the serum-free maintenance mediums of Comparative Examples 7 and 8, in which dexamethasone and insulin were excluded, Fabp4 specific for overall adipocytes was expressed to some extent, but Ucp1 is hardly expressed.

Thus, in addition to rosiglitazone, it was shown that the combination of dexamethasone, insulin, linoleic acid-oleic acid-albumin, and penicillin/streptomycin was preferred as components of the maintenance medium for brown adipocytes.

### [Test Example C] Evaluation of Composition or Medium according to the Present Invention

### (1) Cell culture

The same human fibroblasts as those in Test Example A mentioned above were cultured under the same conditions.

### (2) Differentiation induction from human fibroblasts to brown adipocytes

After the human fibroblasts reached 80% to 90% confluency, the medium in the dish was replaced with SFBAM, a serum-free induction medium, containing all the compounds shown in Table 3 and the human fibroblasts were cultured in the SFBAM for four weeks. The medium was changed every three days. The brown adipocytes obtained at this stage were first subjected to immunostaining (mentioned later) (Example 7).

Subsequently, the induced cells were cultured in a serum-free maintenance medium for brown adipocytes (hereinafter referred to as "SFBAMaM") prepared by adding each of the components shown in Table 7 to a DMEM medium (Cat#: 11965092; manufactured by Gibco Co., Ltd.) for another one week to make the chemically induced brown adipocyte-like cells (ciBAs) mature. The brown adipocytes after maturation were also immunostained (mentioned later) in the same manner (Example 8).

**[Table 7]**

| Components | Final conc. | |
|---|---|---|
| Rosiglitazone (184-02651, by FUJIFILM Wako Pure Chemical Co.) | 1 µmol/L | Selective PPAKγ agonist |
| Dexamethasone (041-18863, by the same company above) | 0.5 µmol/L | Glucocorticoid receptor agonist |
| Insulin (093-06351, by the same company above) | 6.6 µg/mL | |
| Linoleic acid-Oleic acid-Albumin (L9655, by Merck) | 1 mg/mL | Fatty acid-bound albumin |
| Penicillin-Streptomycin (15070063, by Gibco) | 100 U/mL | Antibiotic |

### (3) Immunostaining

Immunocytochemistry was performed as previously described (Dai P, et al. J Clin Biochem Nutr, 2015; 56(3): 166-70). First, the cells were fixed with 4% paraformaldehyde (Nacalai Tesque). Afterwashed with phosphate-buffered saline (PBS) three times, the cells were incubated with PBS containing 0.1% Triton X-100 for 10 min. The cells were then blocked with PBS containing 3% skim milk for 1 hour at room temperature. UCP-1 antibodies (ab10983, Abcam, Cambridge, UK) were diluted 1/500 with blocking solutions. The cells were incubated with the antibodies overnight at 4 °C. After washed with PBS three times, the cells were incubated with Alexa Fluor 488 Donkey anti-Rabbit IgG (A-21206, Thermo Fisher Scientific) for one hour at room temperature. Cell nuclei were stained with DAPI solutions (Dojindo Laboratories). All images were acquired using fluorescent microscopy (Axio Vert.A1, Carl Zeiss, Oberkochen, Germany). Mitochondria were stained using MitoTracker (registered trademark) Red CMXRos (Thermo Fisher Scientific).

### (4) Results

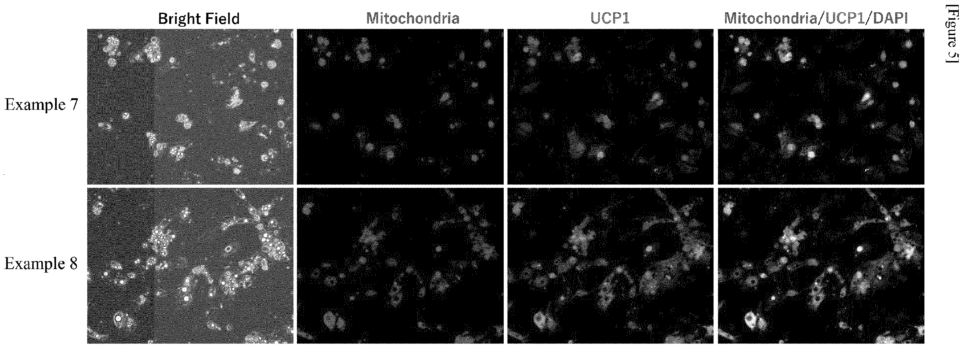

The results of this experiment are shown in Figure 5. As shown in Figure 5, sufficient direct differentiation induction, direct conversion, from human fibroblasts to brown adipocytes was observed in SFBAM, a serum-free induction medium. Further, brown adipocytes with more fat droplets were observed by incubating for one week in SFBAMaM, a serum-free maintenance medium, after induction for four weeks in SFBAM.

### [Test Example D] Investigation of Induction Period to Brown Adipocytes

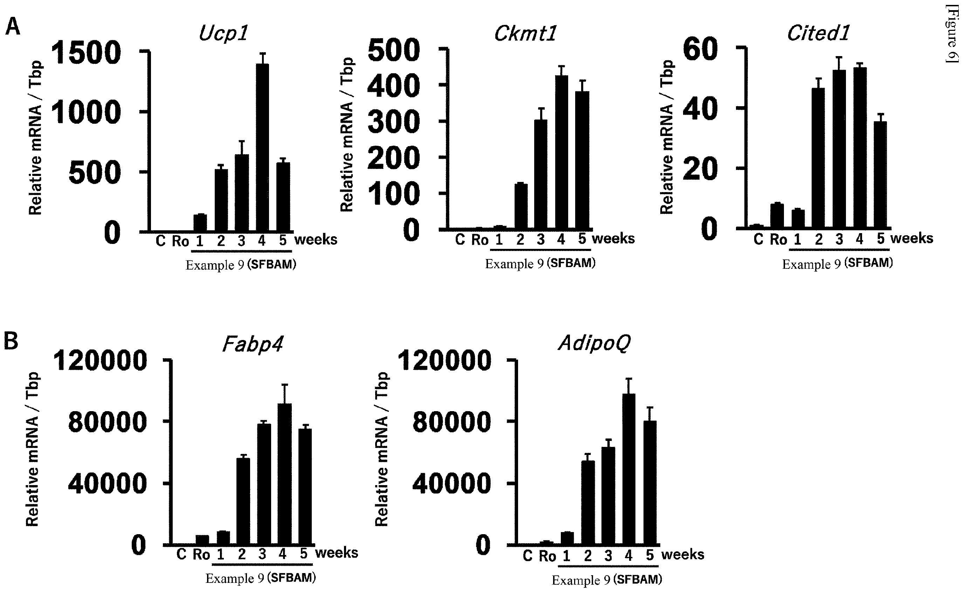

As in Test Example C, after human fibroblasts reached 80% to 90% confluency, the medium in the dish was replaced with SFBAM, a serum-free induction medium, containing all the compounds shown in Table 3 and the human fibroblasts were cultured in the medium, and the culture experiment in SFBAM containing all the compounds shown in Table 3 was designated as Example 9. The medium was changed every three days. Then, the expression amounts of a plurality of marker genes shown in Table 8 below, which are specific for adipocytes and brown adipose tissue, were quantified by QRT-PCR. Figure 6 shows these results. In the figure, the mark "C" shows a case of culturing for five weeks in the same manner but without the compounds shown in Table 3, which are necessary for induction into brown adipocytes, and the mark "Ro" shows a case of culturing for five weeks in the same manner with only rosiglitazone among the compounds shown in Table 3. Note that the genes shown in Figure 6A are specific to brown adipocytes, and the genes shown in Figure 6B are specific to overall adipocytes.

**[Table 8]**

| Genes | Sense primer | Antisense primer |
|---|---|---|
| *Tbp* | ACTACGGGGTTATCACCTGTGAG (Sequence No.1) | GTGCAGGAGTAGGCCACATTAC (Sequence No.2) |
| *Ucp1* | TCTACGACACGGTCCAGGAG (Sequence No.3) | GAATACTGCCACTCCTCCAGTC (Sequence No.4) |
| *Fabp4* | GCCAGGAATTTGACGAAGTCA (Sequence No.5) | CCCATTTCTGCACATGTACCAG (Sequence No.6) |
| *Ckmtl* | AGCAGGAATGGCTCGAGAC (Sequence No.7) | ATCCTCCTCATTCACCCAGATC (Sequence No.8) |
| *Cited1* | TGGCACCTCACCTGCGAAG (Sequence No.9) | GCAGAATGGCCACTGCTTTG (Sequence No.10) |
| *AdipoQ* | CTGGTGAGAAGGGTGAGAAAG (Sequence No.11) | GTTTCACCGATGTCTCCCTTAG (Sequence No.12) |

As shown in Figure 6, if all the compounds shown in Table 3 were contained, all the genes started to be expressed by the first week of culture, and the expression level continued to increase until the third week to the fourth week. In consideration of this result, it is considered that the brown adipocytes induced to differentiate from fibroblasts by the present invention begin to appear by the first week and continue to increase until the third week to the fourth week.

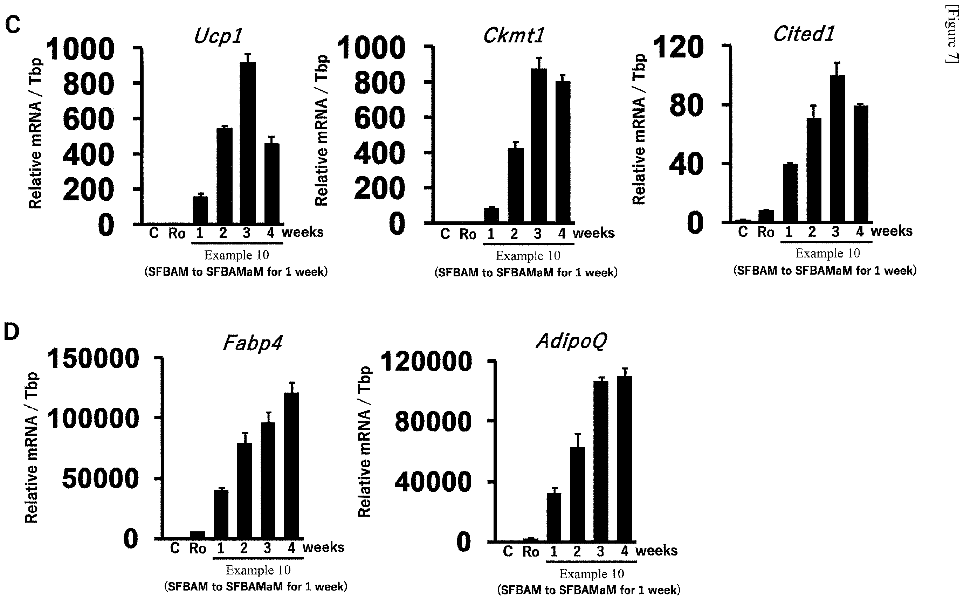

Subsequently, the medium was changed to SFBAMaM, the present invention maintenance medium, and the induced cells were matured for one week. The maintenance culture experiment in this SFBAMaM was designates as Example 10. The results were shown in Figure 7. The marks "C" and "Ro" in the figure referred to the same meaning as those in Figure 6, but the medium was similarly changed to SFBAMaM and the cells were cultured for one week. Likewise, the genes shown in Figure 7C were specific for brown adipocytes, and the genes shown in Figure 7D were specific for overall adipocytes.

As shown in Figure 7, after maturation in SFBAMaM for one week, brown adipocytes induced in SFBAM containing all the compounds shown in Table 3 were maintained with minimum medium components, without a new induction of brown adipocytes and without a great reduction in the expression levels of genes specific for brown adipocytes and adipocytes.

### [Test Example E] Investigation of the Presence or Absence of Fatty Acids in Serum-Free Medium

After the human fibroblasts reached 80% to 90% confluency, the medium in the dish was replaced with SFBAM which is serum-free or SFBAM in which albumin and fatty acid components were changed to the combinations shown in Table 10 of the compounds shown in Table 9, both of which contain all the compounds shown in Table 3, and the human fibroblasts were cultured in said respective media for three weeks (Examples 11 to 14). The medium was changed every three days. Thereafter, the expression amounts of marker genes, Ucp1 and Ckmt1, specific for brown adipose tissue were quantified by QRT-PCR. The results are shown in Figures 8 and 9. Symbol 1 (control) in the figures represents the result in SFBAM without the compounds shown in Table 3 necessary for induction into brown adipocytes.

**[Table 9]**

| Compounds | Final conc. |
|---|---|
| Linoleic acid-Oleic acid-Albumin (L,O-Alb) (L9655, by Merck) | 1 mg/mL |
| Fatty acid-free albumin (FF-BSA) (017-15146, by FUJIFILM Wako Pure Chemical Co.) | 1 mg/mL |
| Oleic acid (155-03401, by the same company) | 20 µmol/L |
| Palmitic acid (165-00102, by the same company) | 20 µmol/L |

**[Table 10]**

| Components | Example 11 | Example 12 | Example 13 | Example 14 |
|---|---|---|---|---|
| L,O-Alb | + | - | - | - |
| FF-BSA | - | + | + | + |
| Oleic acid | - | - | + | - |
| Palmitic acid | - | - | - | + |

In Table 10, the mark "+" indicates that the component was added to the medium, and the mark "-" indicates that the component was not added to the medium.

As shown in Figures 8 and 9, induction under fatty acid-free conditions showed higher expression of marker genes specific for brown adipose tissue.

### INDUSTRIAL APPLICABILITY

The present invention induction medium (e.g., SFBAM) is useful as a medium for differentiation induction from human fibroblasts to brown adipocytes, and the present invention maintenance medium (e.g.,

## Claims

1. A composition for a differentiation induction medium used in differentiation induction from somatic cells to brown adipocytes, comprising the following seven components:
• a thyroid hormone receptor agonist
• a glucocorticoid receptor agonist
• a phosphodiesterase inhibitor
• insulin
• an ascorbic acid derivative
• albumin, and
• an antibiotic.

2. The composition for a differentiation induction medium according to Claim 1, wherein the differentiation induction is performed directly without introducing a gene into a somatic cell.

3. The composition for a differentiation induction medium according to Claim 1 or 2, wherein the thyroid hormone receptor agonist is triiodothyronine, the glucocorticoid receptor agonist is dexamethasone, the phosphodiesterase inhibitor is isobutylmethylxanthine, the ascorbic acid derivative is L-ascorbic acid 2-phosphate, the albumin is an albumin to which linoleic acid and oleic acid are bound or a fatty acid-unbound albumin, or the antibiotic is penicillin and/or streptomycin.

4. The composition for a differentiation induction medium according to any one of Claims 1 to 3, further comprising a selective PPAKγ agonist, or a selective PPAKγ agonist and a cAMP inducer and/or a BMP7.

5. The composition for a differentiation induction medium according to Claim 4, wherein the selective PPAKγ agonist is rosiglitazone or cAMP inducer is forskolin.

6. A differentiation induction medium, comprising the composition for a differentiation induction medium according to any one of Claims 1 to 5, and being serum-free.

7. The differentiation induction medium according to Claim 6, prepared by adding the composition for a differentiation induction medium according to any one of Claims 1 to 5 to a DMEM medium.

8. The differentiation induction medium according to Claim 7, wherein the DMEM medium is a high glucose DMEM medium.

9. The differentiation induction medium according to Claim 7 or 8, wherein the DMEM medium contains L-glutamine.

10. The composition for a differentiation induction medium or the differentiation induction medium according to any one of Claims 1 to 9, wherein the somatic cell is a fibroblast.

11. A brown adipocyte produced by using the composition for a differentiation induction medium or the differentiation induction medium according to any one of Claims 1 to 10.

12. A composition for a maintenance medium for maintaining brown adipocytes produced by differentiation induction from somatic cells, comprising the following five components:
• a selective PPAKγ agonist
• a glucocorticoid receptor agonist
• insulin
• albumin, and
• an antibiotic.

13. The composition for a maintenance medium according to Claim 12, wherein the selective PPAKγ agonist is rosiglitazone, the glucocorticoid receptor agonist is dexamethasone, the albumin is an albumin to which linoleic acid and oleic acid are bound or a fatty acid-unbound albumin, or the antibiotic is penicillin and/or streptomycin.

14. A maintenance medium, comprising the composition for a maintenance medium according to Claim 12 or 13, and being serum-free.

15. The maintenance medium according to Claim 14, prepared by adding the composition for a maintenance medium according to Claim 12 or 13 to a DMEM medium.

16. The maintenance medium according to Claim 15, wherein the DMEM medium is a high-glucose DMEM medium.

17. The maintenance medium according to Claim 15 or 16, wherein the DMEM medium contains L-glutamine and pyruvate.

18. The composition for a maintenance medium or the maintenance medium according to any one of Claims 12 to 17, wherein the somatic cell is a fibroblast.
